Europäisches Patentamt

European Patent Office

Office européen des brevets

(11) Publication number: **0 000 654**

**A1**

(12) **EUROPEAN PATENT APPLICATION**

(21) Application number: **78300182.9**

(22) Date of filing: **21.07.78**

(51) Int. Cl.²: **A 61 M 5/14**

(30) Priority: **25.07.77 US 818629**

(43) Date of publication of application:
**07.02.79 Bulletin 79/3**

(84) Designated contracting states:
**BE CH DE FR GB LU NL SE**

(71) Applicant: **Merck & Co., Inc. Patent Department
126 East Lincoln Avenue Rahway,
N.J. 07065. (US)**

(72) Inventor: **Miller, Neil Warrenier
171-A-3 RD1 Pennsburg
Pennsylvania. (US)**

(74) Representative: **Crampton, Keith John Allen et al
D YOUNG & CO
9 & 10 Staple Inn
London WC1V 7RD. (GB)**

(54) Suspension device.

(57) An inexpensive device for suspending articles generally, and particularly for parenteral, especially intravenous administration of medicament solutions or suspensions, which comprises an article, or particularly a container, especially made of glass, (1), for dispensing medicament solutions or suspensions from an inverted position, and a suspension device (3) stuck to the article, or to the container (1) at the end opposite to that from which the medicament solutions or suspensions are dispensed. The suspension device (3) comprises a pressure-sensitive adhesive layer (8) by means of which the suspension device (3) is stuck to the article or container (1), and, affixed to the opposite side of the adhesive layer (8), a unitary support layer (6) having a configuration that will engage a support device (2) from which the article or container (1) is to be suspended.

Fig. 1

TITLE OF THE INVENTION

Suspension Device

BACKGROUND OF THE INVENTION

The present invention is concerned with a suspension device suitable for adhering to an article for the purpose of suspending said article from a support device.

Particularly, the present invention is concerned with a suspension device suitable for adhering to a container used for dispensing solutions or suspensions, for the purpose of suspending said container from a support device.

The present invention is also concerned with a device for dispensing solutions or suspensions, and particularly with a device for parenteral, especially intravenous administration of medicament solutions or suspensions.

More particularly, the present invention is concerned with the parenteral, especially intravenous administration of various medicament solutions or suspensions from containers which are inverted during dispensing and are suspended from a support device by means of a suspension device adhered to the end of the container opposite to the end from which the medicament solutions or suspensions are dispensed.

The improved suspension device of the present invention is very inexpensive, may be readily and semipermanently adhered to a wide variety of articles, including containers, especially glass containers, particularly those for dispensing medicament solutions or suspensions, and has a configuration which will engage a support device from which the article or container is to be suspended.

The art related to devices for suspending articles, particularly containers, especially those employed in dispensing medicament solutions or suspensions, is replete with a variety of such devices, which exhibit different types of suspension techniques. For example, the suspension device may be formed as an integral part of a container from which some liquid is to be dispensed. U.S. Patent No. 2;698,619 describes a flexible bag with an opening and fitted therein a grommet for suspending the bag from a hook. See also U.S. Patent Nos. 3,171,412; 3,482,572; and 3,583,460. It will be noted that the disclosures of all of these patents concern containers that are fabricated from flexible plastic materials.

Other suspension devices involve the permanent attachment of the device, to a container. For example, see U.S. Patent No. 3,153,414, which describes attachment of a handle to the inner wall of a flexible container by means of dielectric, thermal, or chemical sealing. See also U.S. Patent Nos. 2, 328,569 and 2,409,734.

Yet another type of suspension device is that which serves as a separate holder for a container from which some liquid is to be dispensed, contacting the container, but without being affixed or otherwise attached to the container. See, for example, U.S. Patent No. 2,362,523 which describes a suspension member comprising a base plate of ductile material having a hook formed therefrom; U.S. Patent No. 2,579,701 which describes an infant's bottle holer; U.S. Patent Nos. 3,366,360, 3,395,882 and 3,865,339 which describe harness-like bottle holders; U.S. Patent No. 3,402,910 which describes a disposable bottle holder; U.S. Patent No. 3,807,679 which describes a one-piece molded bottle band; U.S. Patent No. 3,893,495 which describes a bag-like bottle holder; and U.S. Patent No. 3,913,879 which describes a bottle holder comprising a tubular body of mesh construction.

Other types of suspension devices require some type of modifiction of a container which is to be suspended in order to assure secure fastening of the suspension device to the container. See, for example, U.S. Patent Nos. 2,825,521; 3,311,268; and 3,638,822.

The suspension device of the present invention differs from suspension devices hiterto employed in the art in not having to be an integral part of the article or container to be suspended, or in not having to be permanently attached to the article or container, or in not requiring some modifiction of the article or container in order to assure secure fastening of the suspension device to the article or container, or in not involving some elaborate configuration which encompases most, or a significant part of the article or container which is to be suspended. The suspension device of the present invention is, accordingly, of simple construction and of very little cost, yet reliable and easy to employ.

The suspension device of the present invention also possesses particular advantages with respect to containers made of glass. Typically, such containers in the past have required special construction in order to accommodate suspension devices of the type described in U.S. Patent No. 3,638,822; or otherwise, suspension devices encompassing most of the glass container have been employed. By contrast, the suspension device of the present invention is adhered only to the end portion of the container, and no special construction features for the container are required.

SUMMARY OF THE INVENTION

The present invention provides a suspension device for adhering to an article, particularly a container, especially one used for parenteral, administration of medicament solutions or suspensions, whereby the article or container may be suspended from a support device.

The term "article" is intended to broadly describe any of a wide variety of pieces of goods to which the suspension device of the present invention might be adhered where it would be desirable to suspend said piece of goods. For example, items for sale in a retail establishment would be readily placed on display with the aid of the suspension device of the present invention.

Likewise, the term "container" is intended to be broadly descriptive of a wide variety of receptables in various shapes and sizes and manufactured from a number of different materials.

The term "medicament solutions or suspensions" is intended to include all liquids normally dispensed during the course of some therapeutic treatment, and is understood to include conventional intravenous saline and glucose solutions as well as solutions or suspensions of variuos drugs which are intended for intravenous, or some other form of parenteral administration.

The container from which the medicament solution or suspension is to be dispensed may be, basically, of any construction configuration and may be made, essentially, of any material. Thus, the container may be of the usual configuration employed for glass bottles used to dispense various pharmaceutical liquids. The container may also be, on the other hand, of the typical bag-like configuration associated with use of flexible plastic materials in construction.

The suspension device of the present invention is adhered to an article, particulary a container, for the purpose of suspending the article or container from a support device. The support device may be any means, such as a pole, peg, hook, or the like, whereby the article or container, with the aid of the adhered suspension device, is suspended from the support device. The purpose served by suspending the article or container may be that of storage, display, gravity-assisted dispensing of a liquid from a container, or some other purpose.

The suspension device of the present invention is adhered to a container from which medicament solutions or suspensions are to be dispensed, for the purpose of suspending the container from the arm of a support device, whereby the medicament solution or suspension may be continually dispensed, usually by gravity flow.

Such support devices for use in administering medicament solutions or suspensions are well known and typically include an upright pole supported by a platform stand. The pole in turn carries one or more arms. From these arms containers for dispensing medicament solutions or suspensions are hung.

The suspension device of the present invention comprises, first, a pressure sensitive adhesive layer. The composition from which this adhesive layer is made may be selected from a number of natural and synthetic resin materials which would suggest themselves as suitable to the artisan. Particularly, polyester resins and synthetic rubber latex materials have been found especially suitable for making the adhesive layer. The adhesive composition is made to adhere to the surface of the container to which it is to attach the suspension device of the present invention, by the application of pressure. The adhesive composition then becomes semi-permanently bonded to the container surface, i.e., the bond will fail before destruction or separation of the article or container surface and before separation or delamination of the adhesive layer from the support layer of the suspension device. The support layer of the suspension device will be described hereinafter.

The composition of the pressure sensitive adhesive must possess sufficient adhesive strength to adhere satisfactorily and reliably to the surface of the article or container and it must have sufficient cohesive strength to support the weight of the article or container and of the contents of the container, particularly of a solution or suspension being dispensed, for an extended period of time. Of course, it will be appreciated that the extent of the adhesive layer, i.e., its total area, will also be determinative of the weight which the suspension device of the present invention will be able to support. Nevertheless, it would, in any case, be desirable to minimize the size of the suspension device, and, thus, the adhesive and cohesive strength of the pressure sensitive adhesive composition remain important. The adhesive compositions described herein have been found to possess these required characteristics. Other adhesive compositions which would be suitable would suggest themselves to the skilled artisan.

The suspension device of the present invention comprises, second, a unitary support layer. The term "unitary" describes the particular advantageous feature of the suspension device of the present invention that it is formed from a single thickness of material, without separate interconnected parts, and is thus characterized by simplicity and low cost.

The support layer of the suspension device of the present invention must have a configuration which will engage a support device, particularly the arm of a support device used to administer medicament solutions or suspensions. A number of such configurations are possible.

The term "unitary" also describes a preferred configuration of the suspension device of the present invention wherein the support layer comprises a horizontal portion and a vertical portion. These portions are made from the unitary support layer by cutting, stamping, or in some other way separating the two portions from each other on the support layer. The separation is complete except for one side by which the vertical portion remains hingingly attached to the horizontal portion. Initially, at the time the suspension device is made, the horizontal portion and the vertical portion are coplanar. As a result of the one unseparated side by which the two portions are hingingly attached, the vertical portion is adapted to be moved at an angle to the horizontal portion, whereby it is able to engage a support device and thereby suspend the article or container, especially a container from which medicament solutions or suspensions are to be dispensed. Thus, the terms "horizontal" and "vertical" describe the relative positions of the two portions of the unitary support layer when the suspension device of the present invention is in use, adhered to the bottom of some article, particularly a bottle container which has been inverted for the purpose of dispensing medicament solutions or suspensions therefrom.

In the particular configuration just described, the vertical portion of the support layer is provided with an opening or aperture to engage the support device, particularly, to accommodate the arm of a support device used to administer medicament solutions or suspension. This aperture is readily made at the time the horizontal and vertical portions are separated during manufacture of the suspension device. The aperture is most appropriately circular and its diameter is chosen to accommodate conventional support devices and support device arms. The aperture may also be star-shaped with a multiplicity of points which tend to grip and hold the arm of a support device when it is inserted through the aperture.

A particular advantage of the configuration just described is that it permits the horizontal and vertical portions of the support layer to reassume a coplanar relationship, which greatly facilitates storage of containers to which suspension devices of the present invention have been adhered.

The support layer must be made from a material which has sufficient strength, particularly tear-resistance, to provide secure suspension of the article or container, especially a container from which medicament solutions or suspensions are dispensed, for an indefinite period of time. The material must be able to withstand the considerable stress under which it will be placed by movement of the article or container on the support device from which the article or container is suspended, during typical manipulation while in use. Thus, the support material should have a Strip Tensile strength, for a 1 inch strip, of at least 45 lbs/inch Machine Direction (M.D.), and of at least 40 lbs/inch Cross Direction (C.D.) as measured by TAPPI Method T404M50. It has been found that especially suitable materials are those made from high density polyethylene fibers by an integrated spinning and bonding process. Such a material is that available under the trademark Tyvek, from the E.I. DuPont de Nemours Company.

The pressure sensitive adhesive layer and the support layer of the suspension device of the present invention may be coextensive, i.e., the support layer may be uniformly affixed to the pressure sensitive adhesive layer at all points. However, it is also possible to nonuniformly affix the support layer to the pressure sensitive adhesive layer. This would be particularly desirable for the configuration described above where the support layer is separated into horizontal and vertical portions. For such a configuration the pressure sensitive adhesive would be affixed only to the horizontal portion of the support layer. Absence of the adhesive composition from the vertical portion of the support layer would avoid problems during moving of the vertical portion at an angle to the horizontal portion created by the tendency of the adhesive to also adhere the vertical portion to the bottom of the container to be suspended. Such absence would also avoid problems created by subsequent interference of the exposed adhesive with objects with which it might later come in contact.

An alternative embodiment of the suspension device of the present invention provides for inclusion of a layer of flexible material disposed between the pressure sensitive adhesive layer and the support layer. Such a layer of flexible material permits the suspension device of the present invention to readily conform to an irregular surface of an article or container to which it is to be attached. Suitable flexible materials would include, for example, polyurethane foam and synthetic rubber foam compositions. It is possible, of course, to make the support layer itself from such a flexible material, thus avoiding the necessity of a third layer. However, in such a case the flexible material must possess sufficient strength, especially resistance to tearing, to serve as the support layer material.

## BRIEF DESCRIPTION OF THE DRAWING:

In the accompanying drawing, illustrating certain preferrred embodiments of the present invention, the same reference numerals designate the same parts of all of the views:

FIG. 1 is a perspective view of a suspension device of the present invention adhered to an inverted container and engaging the arm of a support device (not shown);

FIG. 2 is an enlarged cross-sectional view of a suspension device containing a layer of flexible material;

FIG. 3 is a crosssectional view of the suspension device containing a layer of flexible material adhered to a container;

FIG. 4 is a fragmentary perspective view of a suspension device containing two vertical portions;

FIG. 5 is a fragmentary perspective view of a suspension device in which the horizontal portion covers a limited portion of the bottom of a container to which it is applied;

FIG. 6 is a fragmentary perspective view of a suspension device where the horizontal portion has tabs adhered to the bottom sidewall of a container, in addition to covering the bottom of the container.

## DESCRIPTION OF THE PREFERRED EMBODIMENTS

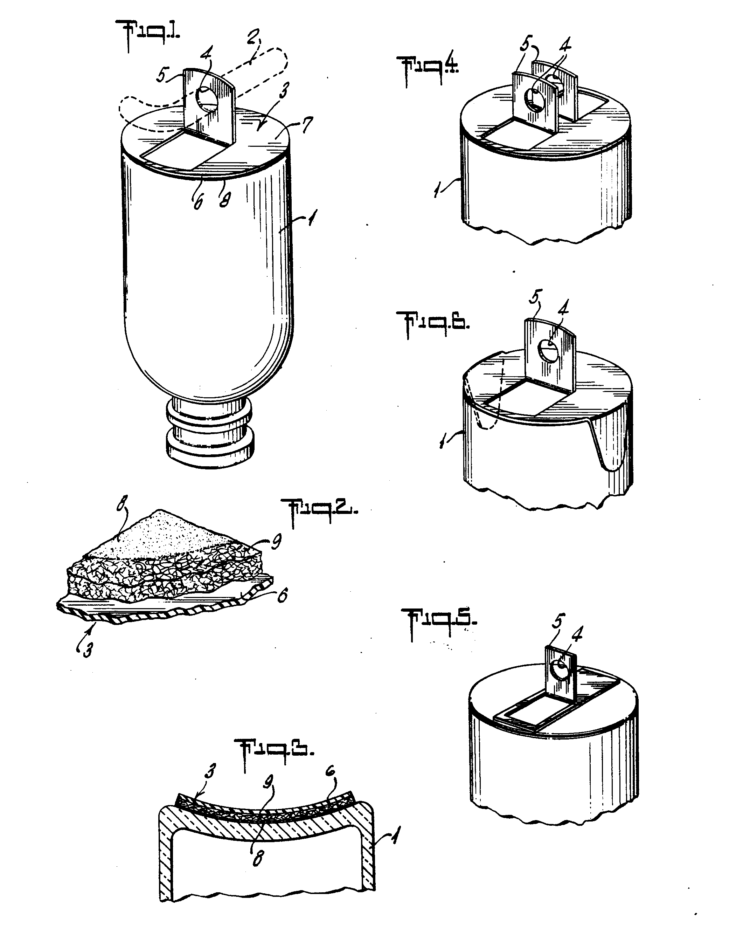

Referring more particularly to the drawing, in FIG. 1 container 1, from which medicament solutions or suspensions are to be dispensed, is suspended from the arm 2 of a support device (not shown) by means of suspension device 3. The arm 2 is engaged by passing through aperture 4 in vertical portion 5 of support layer 6. Horizontal portion 7 comprises the remainder of support layer 6. Suspension device 3 also comprises pressure sensitive adhesive layer 8. It will be noted that the suspension device is adhered to the entire bottom surface of container 1.

FIG. 2 illustrates the inclusion of a third layer of flexible material 9 disposed between pressure sensitive adhesive layer 8 and support layer 6. FIG. 3 is a crosssectional view of such a suspension device adhered to the concave bottom of a container for dispensing medicament solutions or suspensions. It can be seen that the layer of flexible material permits the suspension device to more readily conform to the irregular surface of the container.

FIG. 4 illustrates the use of two vertical portions of the support layer disposed parallel to each other with their apertures aligned so that they both will engage the arm of a support device. This configuration results in noticeably improved stability with respect to resistance of the suspended container and suspension device to swaying i.e., rotation about the arm of the support device.

FIG. 5 illustrates the use of a suspension device of reduced size which covers only a limited portion of the bottom of the container to which it is adhered. Such a suspension device would be useful where it was desired to suspend containers and their contents having total weights that were smaller than usual. By contrast, FIG. 6 illustrates a suspension device that not only covers the entire bottom area of the container to which it is adhered, but which also comprises tabs or extensions which are adhered as well to the bottom sidewall of the container. This suspension device is clearly able to support a greater total weight of a container and its contents than the suspension device illustrated in FIG. 5. It is clearly a matter of ordinary skill and routine experimentation to determine the size of suspension device required to satisfactorily suspend a given total weight of a container and its contents.

The suspension device of the present invention may be manufactured by any suitable means within the skill of the ordinary artisan instructed by the description herein as to the requirements for the finished article. For example, a continuous strip or band of material suitable for the support layer may first have applied thereto in a uniform application, or in a nonuniform pattern, a suitable pressure sensitive adhesive composition. There may then be applied to the adhesive composition a material having a release coating or an abhesive material, i.e., a material which will only lightly adhere to the adhesive composition and which may be later removed therefrom easily, thus permitting handling of the composite laminate of materials. The continuous composite laminate thus created may then be cut by a stamping die into the desired configuration for the suspension device, and the individual suspension devices then separated and collected. These suspension devices may then be distributed for eventual use with articles or containers to be suspended, on a one-by-one basis. The suspension devices may also be immediately applied to articles or containers which are to be distributed for future use, in which case such articles or containers will be capable of being suspended from a support device without the necessity of installing the suspension device. The suspension device may be applied to an article or container simply by removing the abhesive or release layer and pressing the device firmly against the article or container with its pressure sensitive adhesive side inward.

15

## CLAIMS

1.        A suspension device (3) suitable for adhering to an
article (1) to be suspended, for the purpose of suspending the
article (1) from a support (2), comprising in combination a
pressure-sensitive adhesive layer (8) and, affixed to one side of
the said adhesive layer (8), a unitary support layer (6) having a
configuration that will engage the said support (2).

2.        A suspension device (3) suitable for adhering to a
container (2) used for dispensing solutions or suspensions, at the
end of the container opposite to that from which the solutions or
suspensions are dispensed, for the purpose of suspending the container (1) from a support (2), comprising in combination a pressure-
sensitive adhesive layer (8) and, affixed to one side of the said
adhesive layer (8), a unitary support layer (6) have a configuration that will engage the said support (2).

3.        A suspension device (3) suitable for adhering to a
container (1) used for parenteral and especially intravenous
administration of medicament solutions or suspensions, at the end
of the container opposite to that from which the medicament
solutions or suspensions are dispensed, for the purpose of suspending the said container (3) from the arm of a support (2),
comprising in combination a pressure-sensitive adhesive layer (8)
and, affixed to one side of the said adhesive layer (8), a unitary
support layer (6) having a configuration that will engage the said arm of the support (2).

4.      A suspension device as claimed in any one of Claims 1 to 3 in which the configuration of the support layer comprises a horizontal portion (7) affixed to the adhesive layer (8) and a vertical portion (5) cut from the said horizontal portion (7) except for one side by which it remains hingedly attached to the said horizontal portion, the vertical portion (5)(a) being coplanar with the horizontal portion but movable at an angle to it when it is desired to suspend a container (1) by the device, and (b) having an aperture (4) through it to engage the support (2).

5.      A device as claimed in Claim 4 in which the aperture (4) is a circular opening.

6.      A device as claimed in Claim 4 or 5 in which two vertical portions (5) are cut from the horizontal portion (7) and are disposed in parallel planes, and the apertures (4) through them are aligned to engage the support (2).

7.      A device as claimed in any one of Claims 1 to 6 additionally including a layer of flexible material (9) disposed between the adhesive layer (8) and the support layer (6) whereby the device may be readily made to conform to an irregular surface of a container (1) to which it is to be attached.

8.      A device as claimed in any one of Claims 1 to 7 in which the support layer (6) is composed of a material having a Strip Tensile strength, for a 1-inch (2.5-cm.) strip, of at least 45 lbs/ inch M.D. and 40 lbs/inch C.D., (20 kg per 2.5 cm M.D. and 18 kg per 2.5 cm C.D.) as measured by TAPPI Method T404M50.

9.      A device as claimed in any one of Claims 1 to 8 in which the support layer (6) is composed of a material made from high-density polyethylene fibers by an integrated spinning and bonding process.

10.    Apparatus for dispensing solutions or suspensions comprising a container (1) for dispensing the solutions or suspensions and a suspension device (3) stuck to the container (1) at the end opposite to that from which the solutions or suspensions are dispensed, in which the suspension device (3) comprises a pressure-sensitive adhesive layer (8) by means of which the suspension device is stuck to the container, and, affixed to the opposite side of the adhesive layer (8), a unitary support layer (6) having a configuration that will engage a support device (2) from which the container (1) is to be suspended.

11.    Apparatus as claimed in Claim 10 in which the container (1) is for parenteral, especially intravenous, administration of medicament solutions or suspensions from an inverted position.

12.    Apparatus as claimed in Claim 11 in which the container is made of glass.

13.    Apparatus as claimed in Claim 10, 11 or 12 in which the suspension device is as claimed in any one of Claims 2 to 9.

0000654

Fig.1.

Fig.2.

Fig.3.

Fig.4.

Fig.5.

Fig.6.

0000654

# European Patent Office

# EUROPEAN SEARCH REPORT

Application number

EP 78 30 0182

## DOCUMENTS CONSIDERED TO BE RELEVANT

| Category | Citation of document with indication, where appropriate, of relevant passages | Relevant to claim |
|---|---|---|
| | <u>DE - A - 2 323 801</u> (FAENSEN KG) <br> * Figure 12; pages 1-8; claims 1,2 * | 1-5, 10-13 |

### CLASSIFICATION OF THE APPLICATION (Int. Cl.²)

A 61 M 5/14

### TECHNICAL FIELDS SEARCHED (Int.Cl.²)

A 61 M 5/14

### CATEGORY OF CITED DOCUMENTS

X: particularly relevant

A: technological background

O: non-written disclosure

P: intermediate document

T: theory or principle underlying the invention

E: conflicting application

D: document cited in the application

L: citation for other reasons

&: member of the same patent family, corresponding document

The present search report has been drawn up for all claims

| Place of search | Date of completion of the search | Examiner |
|---|---|---|
| The Hague | 08-11-1978 | NOESEN |

EPO Form 1503.1 06.78